# EUROPEAN PATENT SPECIFICATION

(11) **EP 2 800 807 B1**
(45) Date of publication and mention of the grant of the patent: **25.03.2020**
(21) Application number: 13704248.7
(22) Date of filing: 04.01.2013
(51) Int. Cl.: C12M 3/00, C12M 1/00, B33Y 80/00

(54) **BIOREACTOR COMPOSED OF WATERTIGHT CHAMBER AND INTERNAL MATRIX FOR THE GENERATION OF CELLULARIZED MEDICAL IMPLANTS**
BIOREAKTOR AUS EINER WASSERDICHTEN KAMMER UND EINER INTERNEN MATRIX ZUR ERZEUGUNG ZELLULARISIERTER MEDIZINISCHER IMPLANTATE
BIORÉACTEUR COMPOSÉ DE CHAMBRE ÉTANCHE À L'EAU ET MATRICE INTERNE POUR LA GÉNÉRATION D'IMPLANTS MÉDICAUX CELLULARISÉS

(30) Priority: 05.01.2012 PT 12106083
(43) Date of publication of application: 12.11.2014
(73) Proprietor: Association for the Advancement of Tissue Engineering and Cell Based Technologies & Therapies (A4TEC) - Associação, 4710-057 Braga (PT)
(72) Inventor: COSTA, Pedro Ferreira da, 4815-495 Caldas de Vizela (PT); MARTINS, Albino Manuel, Pereira, 4820-005 Antime Fafe (PT); VAQUETTE, Cedryck, F-88390 Uxegney (FR); NEVES, Nuno João Meleiro Alves das, 4700-207 Braga (PT); GOMES, Maria Manuela Estima, 4200 Porto (PT); HUTMACHER, Dietmar Werner, Kenmore Hills 4069 (AU); REIS, Rui Luís Gonçalves dos, 4250-242 Porto (PT); MELCHELS, Ferry Petrus Wilhelmus, 3508 GA Utrecht (NL)
(74) Representative: Patentree
(86) International application number: PCT/PT2013/000001
(87) International publication number: WO 2013/103306

(56) References cited:
- WO-A1-2007/136227
- WO-A1-2010/016023
- WO-A2-2011/073261
- JP-A- 2002 335 950
- JP-A- 2003 225 082

## Description

### OBJECT OF THE INVENTION

The present invention relates to a bioreactor composed of a watertight chamber and one or more matrices contained in its interior, which are simultaneously manufactured, whose matrix can be bi-dimensional or three-dimensional, with variable porosity and delineated by simple or complex shapes, is capable of supporting the seeding and culture of cells for, after removal of the watertight chamber, originating cellularized medical implants for the replacement/regeneration of animal or human tissues or organs previously damaged by trauma or disease.

### STATE OF THE ART

In the replacement or regeneration of tissues or organs after physical damage caused by trauma or disease auto-, allo- or xenografts are currently used in clinical practice. However, these medical grafts have limitations such as the low availability of healthy tissue, immunocompatibility problems or cross infection between different animal species. To overcome these restrictions on its use in the current clinic practice, Tissue Engineering emerged as a promising alternative. This strategy includes the development of bi- or three-dimensional porous matrices that support cellular colonization and culture, the *ex vivo* culture of cells and the presence of growth factors that induce cellular growth and/or differentiation. These factors may be used independently, in groups of two or three in order to allow tissue regeneration.

Being the development of bi- or three-dimensional porous matrices a key step in the success of a Tissue Engineering strategy, these matrices must comprise a set of physicochemical properties (e.g. porosity, interconnectivity, roughness, surface area, mechanical properties, hydrophilicity) that allow cellular adhesion, migration, proliferation and differentiation. The maintenance of cells in culture within these bi- or three-dimensional porous matrices can be achieved through the use of static or dynamic culture systems. It has been demonstrated that the culture of cells in an appropriate biochemical environment and in the presence of mechanical stimuli may promote the generation of cellularized medical implants. Therefore, there is a great interest to replicate *in vitro* the physiological environment of a target tissue, through the use of dynamic simulation biomechanics systems, scientifically known as bioreactors. These bioreactors not only provide the cells in culture within bi- or three-dimensional porous matrices with equal concentrations of nutrients and oxygen, but also allow the removal of byproducts resulting from their metabolic activity. Besides these features, bioreactors also allow a more uniform colonization of the bi- or three-dimensional porous matrices by the cells. Therefore, these dynamic culture systems constitute a better quantitative control method over the parameters of cell culture, and can provide an unlimited number of cellularized medical implants.

WO2010/016023 discloses a bioreactor suitable for implant tissue culture, comprising a chamber adapted to receive a matrix supporting tissue culture. The chamber is provided with ports to achieve fluid flow. The bioreactor is provided with a tab to allow removal of the wall of the chamber by peeling the adhered sheet to extract the tissue.

JP2002335950 discloses storage/transportation container for a membrane tissue comprising a liquid-permeable support which can nip the flat membrane-like membrane tissue comprising the biological material from both the lateral sides and hold the membrane tissue in a state keeping its original shape without deteriorating its biological characteristics, and an openable fluid-tight and/or gas-tight container body which can receive at least one support together with a prescribed storing/transporting liquid and maintain the membrane tissue held in the support in a wet state with the storing/transporting liquid.

The present invention aims at the development of cellularized medical implants with improved biomechanical properties, by developing methods of cellular colonization and culture on bi- or three-dimensional porous matrices. Implicit is the development of a bioreactor which contains, in itself, one or more bi- or three-dimensional porous matrices, allowing minimal handling of the cellularized medical implant(s) in order to minimize the risk of contamination during *ex vivo* cell culture.

### DESCRIPTION OF THE INVENTION

The present invention refers to a bioreactor which can be utilized in the colonization and culture of cells into support matrices contained into their interior, originating cellularized medical implants for the substitution/regeneration of animal or human tissues or organs, such as bone, cartilage, skin and muscle.

The bioreactor is mainly composed of one or more matrices, with variable porosity, three-dimensional or bi-dimensional, contained into a unicompartmented or multicompartmented watertight chamber.

The culture chamber as well as the matrix contained in its interior are simultaneously manufactured through a rapid prototyping process, from one or multiple materials. These materials can be biodegradable or not, inert or not, polymeric or composite, transparent, translucent or opaque, such as polycaprolactone (PCL), polylactic acid (PLA) or polyglycolic acid (PGA).

During the manufacture of the bioreactor and its internal matrix an extra water-soluble support material can be used, which is as well deposited during the rapid prototyping process. This material acts only as a support for the deposition of the deposited material or materials during the manufacture of the chamber and its matrix, being afterwards removed by immersion into water or aqueous solution.

The architecture of the chamber and its internal matrix should preferably be designed to be self-supportive, not being necessary the utilization of an additional support material.

The chambers and their inner matrices can be sterilized by several methods according to the materials utilized. They can be sterilized by immersion into ethanol, by exposure to ultraviolet radiation or exposure to ethylene oxide, in case none of the utilized materials show any adverse reactivity, or even by autoclaving, when the utilized materials are sufficiently stable when exposed to high temperatures.

In case the external chamber is prototyped from one or more transparent or translucid materials it becomes even possible to analyze its interior by optical means. In case the internal matrix is also prototyped from transparent or translucid materials the interior of the internal matrix can also be analyzed by optical means.

In order to improve the performance of the internal matrix contained into the chamber it is possible to perform coatings of its surfaces by filling or perfusing the interior of the chamber, through the internal matrix, with solutions or gases able to generate those coatings.

The internal matrix can be designed using as an initial reference simple shapes such as cuboid, cylindrical, tubular or other shapes, or as well from complex shapes obtained from the analysis of tissues or organs by means of computerized tomography or magnetic resonance techniques.

The design of the external chamber is performed having as initial reference the shape of the internal matrix and by extrusion of its external surfaces.

To the external chamber's design are added at least two tubular structures which are intended to connect the interior of the chamber to the exterior and allowing the entry and exit of fluids and/or gases from/to the interior of the chamber. The location of these tubular structures should be determined taking into account the best possible efficiency in draining the fluids inserted into the chamber as well as the improvement of the fluid flow dynamic into the chamber.

The bioreactor can be integrated into a perfusion culture system which allows to circulate culture medium through the interior of the external chamber and through the pores of the bioreactor's internal matrix.

The culture medium is supplied to the chamber through the tubular structures for entry/exit of medium, preferably located at the extremities of the culture chamber, and circulated from and to an aired culture medium reservoir by means of a peristaltic pump.

When integrated into a perfusion culture system, the bioreactor is able to perform continuous perfusion of expansion or differentiation culture medium, improving the mass transport of cells into the previously cellularized matrix. The continuous perfusion of culture medium performed by the culture system is able to act as a stimulus over the cells, being able to regulate or increase the proliferation and differentiation of cells. The perfusion of medium through the cellularized matrices can be performed both unidirectionally or bidirectionally.

After culture, the cellularized matrix can be removed from the interior of the bioreactor by cutting the external chamber in specifically designed locations, using surgical tools.

### BRIEF DESCRIPTION OF THE DRAWINGS

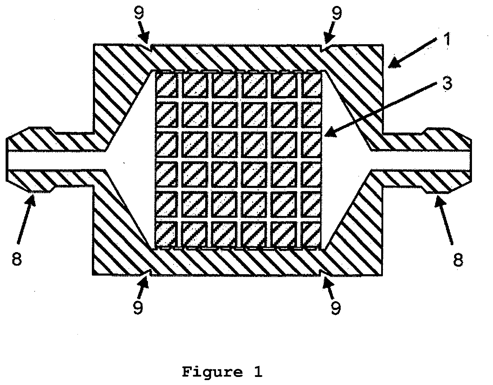

Figure 1 shows a longitudinal section of the device containing a simple three-dimensional porous structure in its interior.

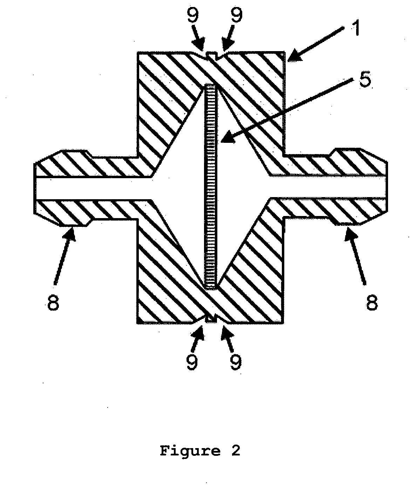

Figure 2 shows a longitudinal section of the device containing a bi-dimensional porous structure in its interior.

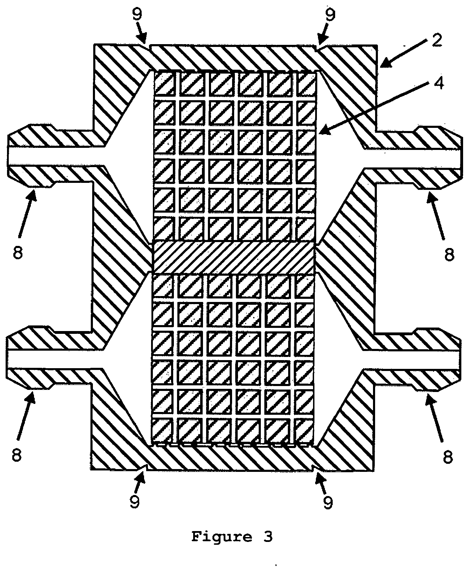

Figure 3 shows a longitudinal section of the device containing a double three-dimensional porous structure in its interior.

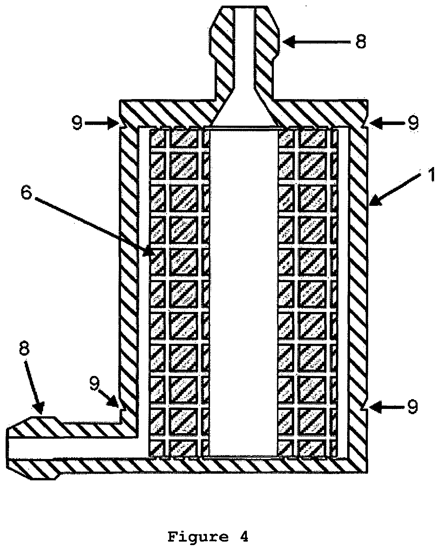

Figure 4 shows a longitudinal section of the device containing a tubular three-dimensional porous structure in its interior.

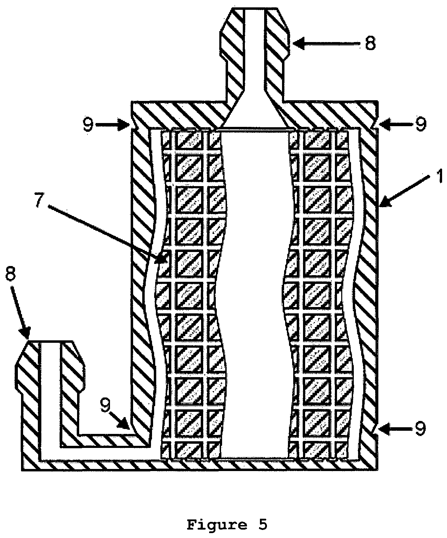

Figure 5 shows a longitudinal section of the device containing a complex tubular three-dimensional porous structure, manufactured using a three-dimensional model obtained from computerized tomography or magnetic resonance analysis of a tissue or organ.

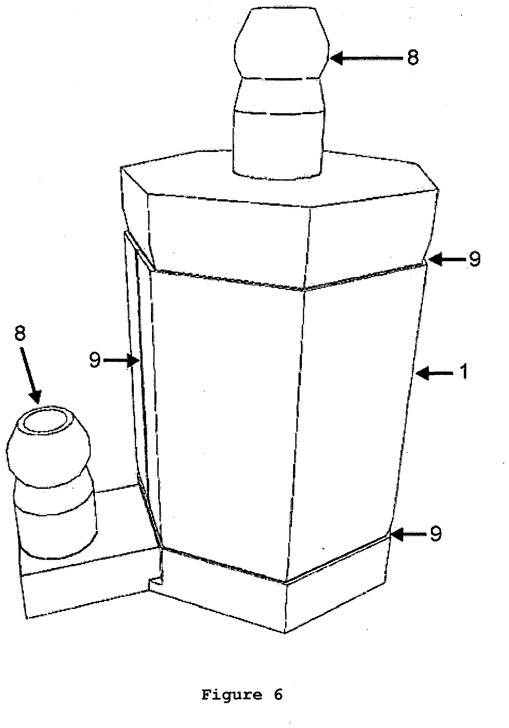

Figure 6 shows the device in isometric view.

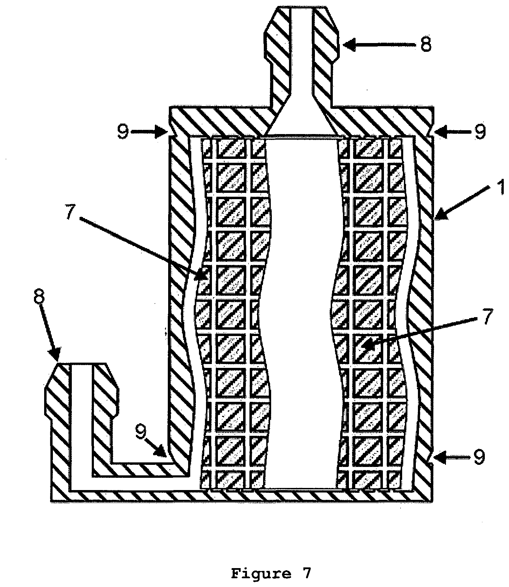

Figure 7 shows a longitudinal sectionof the device.

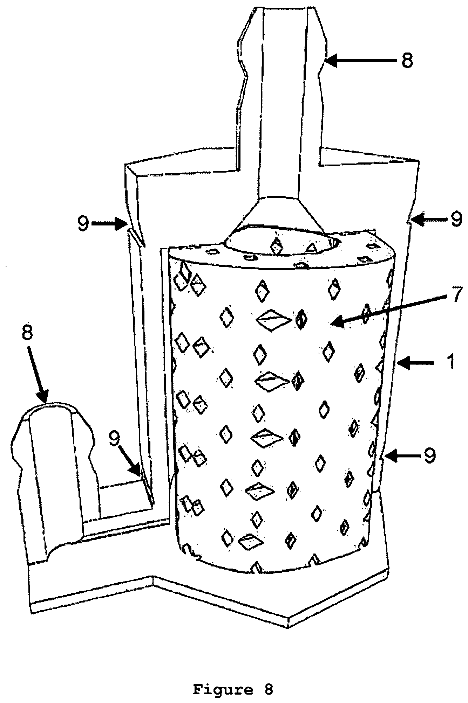

Figure 8 shows a partial section of the device in isometric view.

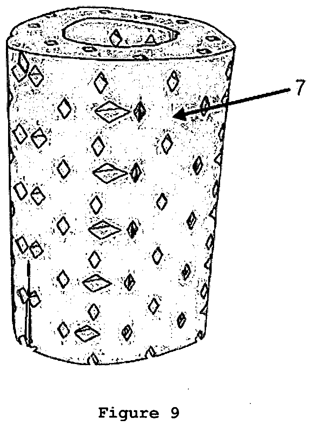

Figure 9 shows an implant after removal of the external enclosure in isometric view.
Figure 10 shows the device described in figures 6 and 9 integrated into a dynamic perfusion cell culture system.

### DETAILED DESCRIPTION OF THE INVENTION

The following description relates to a preferential configuration of the invention resorting to the figures in this document in order to allow a better understanding of the invention.

The bioreactor is essentially composed of one unicompartmented 1 or multicompartmented 2 watertight chambers containing in its interior one or more matrices, possessing porosity which can be uniform or variable, homogenous or heterogenous, present in the whole matrix 3,5,6,7 or part of the matrix 4, three-dimensional 3,4,6,7 or bi-dimensional 5, possessing simple 3, 4, 5, 6 or complex 7 shapes.

The matrix's external simple shape design 3,4,5,6 can be achieved by using simple shapes as initial references such as cuboidal, cylindrical, tubular or other shapes, while the design of the complex 7 internal matrix's external shape is achieved by resorting to complex shapes obtained from analysis performed on tissues or organs using technologies such as computerized tomography or magnetic resonance. The internal matrix's 3,4,5,6,7 internal pores can be generated by manually or automatically adding pores into the internal matrix's 3,4,5,6,7 design or as well later automatically added by defining specific properties for the deposition of material during the conversion of the final three-dimensional design into commands for controlling the equipment which will manufacture the internal matrix 3,4,5,6 by rapid prototyping.

The design of the external chamber 1,2 is in turn performed taking as initial reference the external shape of the internal matrix 3,4,5,6,7 and by modifying, through extrusion of the external faces, a simplified replica of that same internal matrix 3,4,5,6,7, in such a way that the device's designed internal walls 1,2 are very close to the external walls of the internal matrix's design 3,4,5,6,7 but also sufficiently apart so that they do not-touch each other. This feature enables that, after simultaneous prototyping of the internal matrix 3,4,5,6 and external chamber 1,2, the media for cell seeding, culture and differentiation used afterwards in its interior are more efficient in seeding, feeding and stimulating the cells which will adhere to the internal and external surfaces of the internal matrix 3,4,5,6,7 when circulated through them, inside the external chamber 1,2, since all the employed culture medium will be permanently kept in close contact with the cells adhered to the internal and external surfaces of the internal matrix 3,4,5,6,7.

Given this feature, and despite being simultaneously prototyped, the internal matrix 3,4,5,6,7 and external chamber 1,2 are not bound together at any point, facilitating the removal of the internal matrix 3,4,5,6,7 from the interior of the external chamber 1,2 where the cell seeding and culture period took place.

To the external chamber's design 1,2 are preferably added two tubular structures 8 which connect the interior of the chamber 1,2 to the exterior and allow the entry and exit of fluids and gases from/to the interior of the chamber 1,2. The location of these tubular structures 8 should be determined taking into account the best possible efficiency in draining the fluids inserted into the chamber as well as the improvement of the fluid flow dynamic into the chamber 1,2.

To the design of the external chamber 1,2, are also added grooves 9, which will be later used for facilitating its precise cutting in predetermined locations, and so facilitating the removal of the matrix 3,4,5,6,7 from the interior of the external chamber 1,2.

The culture chamber 1,2 as well as the internal matrix 3,4,5,6,7 contained in its interior are simultaneously manufactured, through a rapid prototyping process from one or multiple materials. These materials can be biodegradable or not, inert or not, polymeric or composite, transparent, translucent or opaque.

When employing a rapid prototyping process for manufacturing the bioreactor, which is composed of a culture chamber 1,2 and an internal matrix 3,5,6,7, an extra water-soluble support material can be utilized, which is as well deposited during the prototyping process. This material's sole purpose is to support the deposition of the material or materials from which the chamber and matrix will be made of, and can afterwards be removed by immersion into water or aqueous solution.

Preferably, the architecture of the chamber 1,2 and its internal matrix 3,5,6,7 should be designed in a way to be self supportive, eliminating the need for an additional support material.

When the external chamber is prototyped from one or multiple transparent or translucid materials, it becomes also possible to analyze the interior of the chamber by optical means. If the internal matrix 3,4,5,6,7 located inside the chamber is also prototyped from transparent or translucid materials it becomes also possible to analyze the interior of that matrix 3,4,5,6,7.

Chambers 1,2 and their internal matrices 3,4,5,6,7 can be sterilized by various methods according to the utilized materials. They can be sterilized by immersion into ethanol, by exposure to ultraviolet radiation or to ethylene oxide, in case of absence of any adverse reactivity, or even by autoclaving, in case the utilized materials are sufficiently stable when exposed to high temperatures.

In order to improve the performance of the internal matrix 3,4,5,6,7 contained into the chamber 1,2 it is possible to perform coatings over its surfaces by filling and/or perfusing the interior of the chamber, through the matrix's pores, with solutions or gases which are able to generate those coatings. Additional coatings can also be performed over the chamber's outer walls 1,2 for various purposes, such as increasing the chamber's 1,2 watertightness.

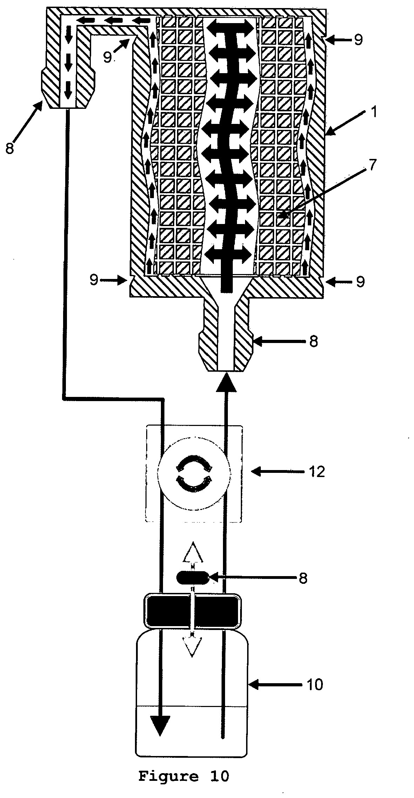

The bioreactor can be integrated into a culture system illustrated in figure 10. This system is composed by the bioreactor in one of its possible configurations, which comprises an external chamber 1,2 containing one of various kinds of internal matrices 3,4,5,6,7, connected to a culture medium reservoir 10 by tubes connected to its tubular structures 8. Apart from the two medium inlets/outlets, the reservoir 10 possesses also an additional connection for the entry and exit of gases which are purified by an air filter 11.

The culture medium is collected from the culture medium reservoir 10, pumped by a peristaltic pump 12 into the external chamber 1,2, perfused through the internal matrix's 3,4,5,6,7 pores and finally pumped by the same peristaltic pump 12 back into the culture medium reservoir 10. This process and circuit is repeated for each individual culture cavity.

For the circulation of culture medium, tubings made from formulations such as silicone should be preferably used since they are highly permeable to gases such as carbon dioxide and oxygen, increasing the gas exchange between circulating medium and surrounding atmosphere.

This culture system can be used not only for culture but as well for the seeding of cells onto the matrix's 3,4,5,6,7 internal and external surfaces for cellular growth. Given its small internal volume this device requires very low volumes of culture medium. For this reason, it is possible to perform dynamic seeding procedures using highly concentrated cell suspensions without using extremely large amounts of cells. In this way, cells have a greater chance to adhere to the matrix's 3,4,5,6,7 internal and external surfaces since they are highly concentrated and are circulated more often through it, making the seeding process more efficient.

When integrated into the perfusion culture system described in figure 10, this bioreactor is capable of improving the cellular mass transport through the cellularized matrix 3,4,5,6,7. The continuous culture medium perfusion provided by the culture system can act as a stimulus over cells, resulting in the regulation or increase of cellular proliferation and differentiation.

Apart from enabling cell seeding and culture by means of a perfusion-based method, this device also allows to use other methodologies such as static or agitated seeding. When performing static seeding, a cell suspension is injected inside the chamber filling all its inner void spaces and in particular the internal matrix's 3,4,5,6,7 pores. After sealing the tubular structures 8 with lids, the cell suspension is kept inside the chamber 1,2 and the matrix's 3,4,5,6,7 pores for a sufficient period of time for cells contained in the cell suspension to adhere to the internal matrix 3,4,5,6,7. During that seeding, the device can be kept in static conditions or as well under agitation in order to increase the possibility for cells to get into contact with multiple matrix's 3,4,5,6,7 surfaces and so increase the possibility of adhesion as well as enabling a more homogenous distribution of adhered cell on the matrix's 3,4,5,6,7 surface.

After the seeding period, the cell suspension containing non adhered cells is removed from the interior of the device and replaced by fresh expansion and/or differentiation medium. Likewise, during the expansion and/or differentiation period, the device containing expansion and/or differentiation medium in its interior can be kept in static conditions or alternatively submitted to agitation.

In order to keep a sterile environment, with stable and adequate temperature and humidity, the bioreactor, integrated or not into a perfusion system, is placed inside a cell culture incubator.

## Claims

1. A bioreactor for the generation of cellularized medical implants **characterized in that** it comprises an external watertight unicompartmented (1) or multicompartmented (2) chamber containing in its interior one or more matrices (3,4,5,6,7), these interior matrices (3,4,5,6,7) and the bioreactor's external chamber (1,2) being simultaneously manufactured through a process of rapid prototyping; wherein the external chamber (1,2) comprises a shape adapted to the implant shape to be cellularized (internal matrix), and **in that** it allows the implants to be removed from the interior of the bioreactor by cutting the external chamber (1,2) in locations comprising grooves (9) right before implantation, enabling the sterility of the implant.

2. A bioreactor according to the previous claim, wherein the culture chamber (1,2) and the internal matrices (3,5,6,7) are auto-supported, although allowing the use of an extra support material, also deposited during the prototyping process, which can afterwards be removed by immersion into water or aqueous solution.

3. A bioreactor according to any of the previous claims, wherein the simple matrix's external shape design (3,4,5,6) is achieved by using simple shapes as initial references such as cuboidal, cylindrical, tubular or other shapes, while the design of the internal complex matrix's (7) external shape is achieved by resorting to complex shapes obtained from tissues or organs analysis performed by technologies such as computerized tomography' or magnetic resonance.

4. A bioreactor according to any of the previous claims, wherein the design of the external chamber (1,2) has as initial reference the external shape of the internal matrix (3,4,5,6,7) in order to the device's designed internal walls (1,2) are sufficiently apart so that they do not touch the external walls of the internal matrix's design (3,4,5,6,7) with a spacing similar to the pore diameter of the inner matrix, which diameter lies preferably between 0.25mm and 3mm.

5. A bioreactor according to any of the previous claims, wherein the internal walls of the external chamber (1,2) are sufficiently apart so that they do not touch the external walls of the internal matrix (3,4,5,6,7) forming a cavity, whose inner filler volume ensures submersion of the internal matrix, by which can be circulated culture media and/or solutions or gases.

6. A bioreactor according to any of the previous claims, wherein it allows to perform coatings over its surfaces by filling and/or perfusing the interior of the chamber, through the pores of the internal matrix, with solutions or gases which are able to generate those coatings.

7. A bioreactor according to any of the previous claims, wherein the external chamber has at least two tubular structures (8) which connect the interior of the external chamber (1,2).

8. A bioreactor according to any of the previous claims, wherein it is manufactured from one or multiple transparent or translucid materials.

9. A bioreactor according to any of the previous claims, wherein the cells/tissues incorporated into the internal matrices (3,4,5,6,7) cultured in the interior of the bioreactor can be submitted to static or dynamic conditions by integration of the bioreactor into a agitation and/or perfusion system.

10. A bioreactor according to any of the previous claims, wherein the internal matrices (3,4,5,6,7) are perfused uni- or bi-directionally.

11. Culture system for the generation of cellularized medical implants wherein it comprises a bioreactor according to any of claims 1-10 for the generation of cellularized medical implants, an external chamber (1,2) containing one of the various kinds of internal matrices (3,4,5,6,7), connected to a culture medium reservoir (10) by tubes connected to its tubular structures (8) and an additional connection for the entry and exit of gases which are purified by an air filter (11).

## Patentansprüche

1. Ein Bioreaktor zur Erzeugung zellularisierter medizinischer Implantate, **dadurch gekennzeichnet, dass** dieser eine äußere wasserdichte ein- (1) oder mehrteilige (2) Kammer umfasst, die in ihrem Inneren eine oder mehrere Matrizen (3, 4, 5, 6, 7) enthält, wobei diese inneren Matrizen (3, 4, 5, 6, 7) und die äußere Kammer (1, 2) des Bioreaktors gleichzeitig über ein Rapid-Prototyping-Verfahren erzeugt werden; wobei die äußere Kammer (1, 2) eine Form umfasst, die an die zu zellularisierende Form des Implantats (innere Matrix) angepasst ist und es dadurch ermöglicht, die Implantate aus dem Inneren des Bioreaktors zu entfernen, indem die äußere Kammer (1, 2) an mit Nuten (9) versehenen Stellen unmittelbar vor der Implantation aufgeschnitten wird, was die Sterilität des Implantats ermöglicht.

2. Ein Bioreaktor nach dem vorhergehenden Anspruch, wobei die Kammer für die Kultur (1, 2) und die internen Matrizen (3, 5, 6, 7) selbsttragend sind, wobei jedoch die Verwendung eines zusätzlichen Trägermaterials möglich ist, das ebenfalls während des Prototyping-Verfahrens eingebracht und anschließend durch Eintauchen in Wasser oder eine wässrige Lösung entfernt wird.

3. Ein Bioreaktor nach einem der vorhergehenden Ansprüche, wobei die einfache Bauform der äußeren Form (3, 4, 5, 6) der Matrix durch die Verwendung einfacher Formen als anfängliche Referenzen, wie quaderförmig, zylindrisch, rohrförmig oder andere Formen, erreicht wird, während die Bauform der äußeren Form der inneren komplexen Matrix (7) durch Rückgriff auf komplexe Formen, erhalten durch die Analyse von Geweben oder Organen mittels Technologien wie Computertomographie oder Magnetresonanz, erreicht wird.

4. Ein Bioreaktor nach einem der vorhergehenden Ansprüche, wobei die Bauform der äußeren Kammer (1, 2) als anfängliche Referenz die äußere Form der inneren Matrix (3, 4, 5, 6, 7) aufweist, sodass die von der Vorrichtung entworfenen Innenwände (1, 2) einen ausreichenden Abstand aufweisen, sodass sie die äußeren Wände der Bauform der inneren Matrix (3, 4, 5, 6, 7) nicht berühren, mit einem dem Porendurchmesser der inneren Matrix ähnlichen Abstand, der bevorzugt zwischen 0,25 mm und 3 mm liegt.

5. Ein Bioreaktor nach einem der vorhergehenden Ansprüche, wobei die Innenwände der äußeren Kammer (1,2) einen ausreichend Abstand aufweisen, sodass sie die Außenwände der inneren Matrix (3,4,5,6,7) nicht berühren, die einen Hohlraum bilden, dessen inneres Füllvolumen das Eintauchen der Innenmatrix sicherstellt, durch den Kulturmedien und/oder Lösungen oder Gase zirkulieren können.

6. Ein Bioreaktor nach einem der vorhergehenden Ansprüche, wobei dieser eine Beschichtung seiner Oberflächen erlaubt, indem das Innere der Kammer durch die Poren der inneren Matrix mit Lösungen oder Gasen, die in der Lage sind, diese Beschichtungen zu erzeugen, gefüllt und/oder perfundiert werden.

7. Ein Bioreaktor nach einem der vorhergehenden Ansprüche, wobei die äußere Kammer mindestens zwei röhrenförmige Strukturen (8) aufweist, die das Innere der äußeren Kammer (1, 2) verbinden.

8. Ein Bioreaktor nach einem der vorhergehenden Ansprüche, wobei dieser aus einem oder mehreren transparenten oder lichtdurchlässigen Materialien hergestellt wird.

9. Ein Bioreaktor nach einem der vorhergehenden Ansprüche, wobei die in die innere Matrizen (3, 4, 5, 6, 7) eingebrachten und im Inneren des Bioreaktors kultivierten Zellen/Gewebe durch die Integration des Bioreaktors in ein Misch- und/oder Perfusionssystem statischen oder dynamischen Bedingungen ausgesetzt werden.

10. Ein Bioreaktor nach einem der vorhergehenden Ansprüche, wobei die inneren Matrizen (3, 4, 5, 6, 7) uni- oder bidirektional perfundiert werden.

11. Kultursystem für die Erzeugung zellularisierter medizinischer Implantate, wobei dieses einen Bioreaktor nach einem der Ansprüche 1-10 zur Erzeugung zellularisierter medizinischer Implantate, eine äußere Kammer (1, 2), die eine der verschiedenen internen Matrizentypen (3, 4, 5, 6, 7) enthält, die mit einem Kulturmediumreservoir (10) durch Rohre mit dessen Röhrenstrukturen verbunden ist, sowie eine zusätzliche Verbindung für den Ein- und Austritt von Gasen, die durch einen Luftfilter (11) gereinigt werden, umfasst.

## Revendications

1. Un bioréacteur pour la génération d'implants médicaux cellularisés **caractérisé en ce qu'**il comprend une chambre étanche à l'eau unicompartimentale (1) ou multicompartimentale (2) contenant en son intérieur une ou plusieurs matrices (3, 4, 5, 6, 7), ces matrices intérieures (3, 4, 5, 6, 7) et la chambre externe du bioréacteur (1, 2) étant simultanément manufacturés à travers un procédé de prototypage rapide ; dans lequel la chambre externe (1, 2) comprend une forme adaptée à la forme d'implant devant être cellularisé (matrice interne), et **en ce qu'**il permet que les implants soient retirés de l'intérieur du bioréacteur en coupant la chambre externe (1, 2) à des emplacements comprenant des rainures (9) juste avant la greffe, permettant la stérilité de l'implant.

2. Un bioréacteur selon la revendication précédente, dans lequel la chambre de culture (1, 2) et les matrices internes (3, 5, 6, 7) sont auto-supportées, bien qu'elles permettent l'utilisation d'un matériau de support extra, également déposé durant le procédé de prototypage, qui peut par la suite être retiré par immersion dans de l'eau ou dans une solution acqueuse.

3. Un bioréacteur selon l'une quelconque des revendications précédentes, dans lequel le design de la forme externe simple de la matrice (3, 4, 5, 6) est atteint en utilisant des formes simples en tant que références initiales, telles que cuboïde, cylindrique, tubulaire ou autres formes, alors que le design de la forme extérieure de la matrice interne complexe (7) est atteint en recourant à des formes complexes obtenues à travers l'analyse de tissus ou d'organes effectuée par des technologies telles que la tomographie informatisée ou la résonance magnétique.

4. Un bioréacteur selon l'une quelconque des revendications précédentes, dans lequel le design de la chambre externe (1, 2) a pour référence initiale la forme externe de la matrice interne (3, 4, 5, 6, 7) afin que les parois internes déssinées du dispositif (1, 2) soient suffisamment espacées afin qu'elles ne touchent pas les parois externes du design de la matrice interne (3, 4, 5, 6, 7) avec un espacement similaire au diamètre de pore de la matrice interne, dont le diamètre se situe entre 0.25mm et 3mm.

5. Un bioréacteur selon l'une quelconque des revendications précédentes, dans lequel les parois internes de la chambre externe (1, 2) sont sufisamment espacées afin qu'elles ne touchent pas les parois externes de la matrice interne (3, 4, 5, 6, 7) formant une cavité, dont le volume de remplissage interne assure la submersion de la matrice interne, par laquelle peuvent circuler des moyens de culture et/ou des solutions ou gaz.

6. Un bioréacteur selon l'une quelconque des revendications précédentes, dans lequel il permet d'effectuer des revêtements sur ses surfaces en remplissant et/ou perfusant l'intérieur de la chambre, à travers les pores de la matrice interne, avec des solutions ou gaz qui sont capables de générer ces revêtements.

7. Un bioréacteur selon l'une quelconque des revendications précédentes, dans lequel la chambre externe a au moins deux structures tubulaires (8) qui connectent l'intérieur de la chambre externe (1, 2).

8. Un bioréacteur selon l'une quelconque des revendications précédentes, dans lequel il est manufacturé à partir d'un ou de plusieurs matériaux transparents ou translucides.

9. Un bioréacteur selon l'une quelconque des revendications précédentes, dans lequel les cellules/tissus incorporés aux matrices internes (3, 4, 5, 6, 7) cultivés à l'intérieur du bioréacteur peuvent être soumis à des conditions statiques ou dynamiques par l'intégration du bioréacteur dans un système d'agitation et/ou de perfusion.

10. Un bioréacteur selon l'une quelconque des revendications précédentes, dans lequel les matrices internes (3, 4, 5, 6, 7) sont perfusées uni- ou bi-directionnellement.

11. Système de culture pour la génération d'implants médicaux cellularisés dans lequel il comprend un biorécteur selon l'une quelconque des revendications 1-10 pour la génération d'implants médicaux cellularisés, une chambre externe (1, 2) contenant un des divers types de matrices internes (3, 4, 5, 6, 7), connecté au réservoir de moyen de culture (10) par des tubes connectés à ses structures tubulaires (8) et une connexion supplémentaire pour l'entrée et la sortie de gaz qui sont purifiés par un filtre d'air (11).
